# EUROPEAN PATENT SPECIFICATION

(11) **EP 2 289 370 B1**
(45) Date of publication and mention of the grant of the patent: **09.05.2012**
(21) Application number: 10167831.6
(22) Date of filing: 30.06.2010
(51) Int. Cl.: A47C 7/00, B60B 33/00

(54) **Displacable chair**
Verschiebbarer Stuhl
Chaise déplaçable

(30) Priority: 27.08.2009 SE 0901132
(43) Date of publication of application: 02.03.2011
(73) Proprietor: Mercado Medic AB, 181 22 Lidingö (SE)
(72) Inventor: Ohlsson, Leif, 18222, Lidingö (SE)
(74) Representative: Strandin, Heléne

(56) References cited:
- EP-A1- 1 360 916
- DE-A1- 3 041 926
- JP-A- 2002 325 652
- US-A- 2 625 989
- US-A- 2 780 277

## Description

### TECHNICAL FIELD

The invention relates chairs for sitting, movable to for positioning of a person.

### BACKGROUND

Movable chairs usually have swiveling castors which enables the chairs to move in all directions in a plane. The construction of the swiveling castors however forces the swiveling castors to rotate around an eccentrically placed, substantially vertical axis, which in turn makes it difficult to control and position the chair with precision, this is a disadvantage when a precise positioning of the person sitting in the chair is needed. Most chairs with swiveling castors further lacks the ability to be adequately brakes against the ground surface, since the braking of the castor wheel usually does not stop the rotation of the swiveling castor around the eccentrically placed substantially vertical axis.

When making x-ray images, one key element is that the x-ray tube and x-ray detector is correctly aligned and positioned in relation to the patient, so that the x-ray image shows the desired part of the body. Furthermore repeatability is desired so that the person analyzing the image knows what the image shows, i.e. which height and angle of the body the image has captured. A key element in maintaining an efficient x-ray room is that the patients can be rapidly positioned in relation to the x-ray tube and x-ray detector and easily be moved to create a series of images showing the body part of interest in a plurality of views.

Advanced x-ray equipment can have functions enabling the x-ray tube and detector to be positioned for the creation of a series of images. However this equipment is usually very large, expensive and requires a great deal of expertise from the personnel operating the equipment, and the positioning of the x-ray tube and detector is usually slow.

Persons whom are injured, elderly or in any other way physically impaired could have a hard time getting to a fixed chair, such as a chair in an x-ray room, especially since the x-ray tube, detector of control electronics could be blocking the access to the chair. The personnel of the x-ray room would like to be able to quickly position the patient in the desired position without any heavy procedures in unpleasant body positions and at the same time ensure repeatability and that the position and/or angle could be documented or traced.

As background art, US2780277 (Ries et al.), US2625989 (Pond et al.), DE3041926 (Lanner), EP1360916 (Glaser) and JP2002325652 (Itoki) all disclose chairs comprising systems for raising and lowering the seat.

### SUMMARY

A chair for sitting is provided. The chair comprises: a first support structure adapted to be movable in relation to the ground surface, a second support structure adapted to have a first and second state, and a seat member. The second support structure is in the first state movable against the ground surface (GS) along with the first support structure. The second support structure is in the second state adapted to display considerable friction against the ground surface. The second support structure is displacably mounted to the first support structure, and the seat member is mounted to the first support structure, such that the seat member is displaceable in relation to the ground surface when the second support structure is in the second state, wherein the second support structure is displacably mounted to the first support structure by a displacement device of said chair. The first and second support structure is connected to the displacement device, and the displacement device is adapted to enable linear movement of the first support structure in relation to the second support structure along a first axis, substantially parallel to the ground surface.

According to one embodiment the first support structure could comprise wheels, and the first support structure could thereby be movable in relation to the ground surface using the wheels. The wheels could according to one embodiment be of swiveling castor type, which enables movement along a plurality of axis of the plane of the ground surface.

According to another embodiment the second support structure is adapted to display considerable friction against the ground surface by friction creating members engaging the ground surface and thereby substantially fixating the chair to a position on the ground surface. For x-ray use, this feature could ensure that the chair remains in a substantially fixated state to the ground surface, which could be an advantage when taking a multiplicity of x-ray images or when analyzing the images.

According to further embodiments the displacement device is further adapted to enable linear movement of the first support structure in relation to the second support structure along a second axis. The chair according to any of the embodiments could also comprise a rotation device adapted to enable rotation of the first support structure in relation to the second support structure, in which case the first and second support structure is directly or indirectly mounted to the rotation device.

The chair could according to one embodiment further comprise a brake which could be adapted to brake any of: the linear movement of the displacement device along the first axis and/or the linear movement of the displacement device along the second axis and/or the rotational movement of the rotation device.

According to some of the embodiments herein, the seat member of the chair further comprises a back rest. In embodiments where the chair is used in x-ray applications the back rest could be transradiant, enabling x-ray images of the person sitting on the seat member to be made without the obstruction of the back rest.

Please note that any embodiment or feature of an embodiment could be combined in any way if such combination is not clearly contradictory.

### BRIEF DESCRIPTION OF DRAWINGS

The embodiments are now described, by way of example, with reference to the accompanying drawings, in which:
Fig. 1a is a schematic figure showing the basic components of a chair according to a first embodiment, in a first state,
Fig. 1b is a schematic figure showing the basic components of the chair according to the first embodiment, in a second state,
Fig. 2 shows a chair according to a second embodiment, in a perspective view,
Fig. 3 shows the chair according to the second embodiment, in a frontal view,
Fig. 4 shows the chair according to the second embodiment, in a side view,
Fig. 5 shows the chair according to the second embodiment, from underneath.

### DETAILED DESCRIPTION

The invention will now be described in more detail in respect of preferred embodiments and in reference to the accompanying drawings. All examples herein should be seen as part of the general description and therefore possible to combine in any way in general terms. Again, individual features of the various embodiments may be combined or exchanged unless such combination or exchange is clearly contradictory to the overall function of the device.

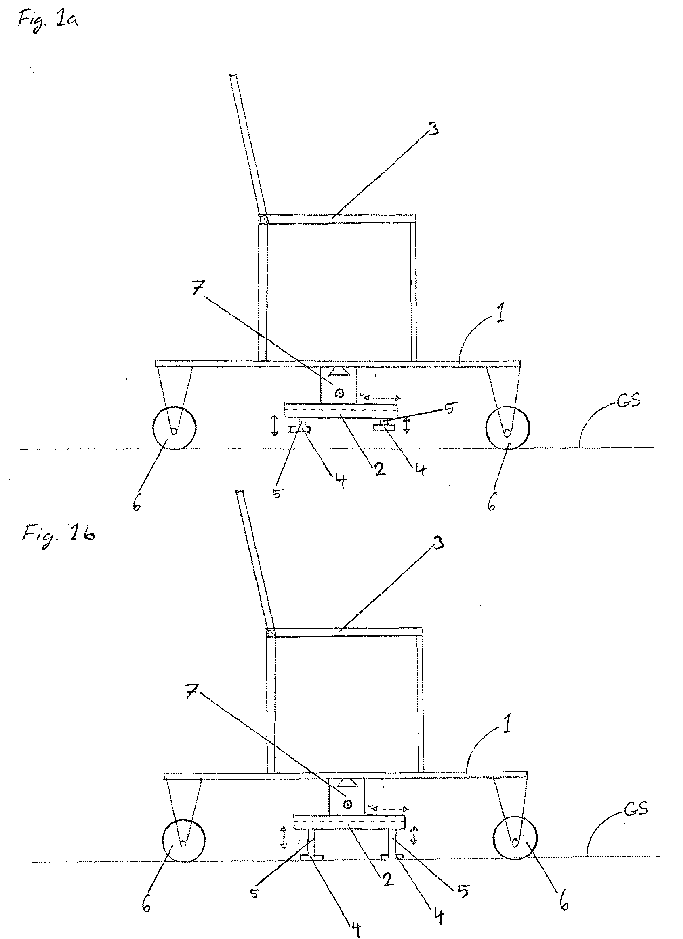

Fig. 1a shows, schematically, an embodiment of a chair in a side view. The first support structure 1 comprises a plurality of wheels 6 which are adapted to be in contact with the ground surface GS for supporting the chair. The wheels 6 are preferably adapted to enable movement along more than one axis. The first support structure 1 is through the wheels 6 adapted to be movable in relation to the ground surface GS. A second support structure 2 is adapted to have a first and second state. In the first state, shown in fig. 1a, friction creating members 4 are not in contact with the ground surface GS by means of actuation devices 5 keeping the friction creating members 4 elevated. The chair further comprises a seat member 3 on which a person could be seated. The second support structure 2, in the first state, is movable in relation to the ground surface GS along with the first support structure 1, since the friction creating members 4 are elevated in the first state.

Fig. 1b shows the chair according to the embodiment of fig. 1a when the second support structure 2 is in its second state. The second support structure 2 now displays considerable friction against the ground surface GS, due to the actuators 5 pressing the friction creating members 4 against the ground surface GS. In this second state, the seat member 3 mounted to the first support structure 1 is still displaceable in relation to the ground surface GS by means of the displacement device 7, which according to this embodiment is adapted to enable displacement along two axis.

The second support structure 2 is displacably mounted to the first support structure 1 by means of a displacement device 7 which is the part connecting the first support structure 1 to the second support structure 2. The second support structure 2 comprises the two actuation devices 5 which actuates the friction creating members 4 and thereby transfers the second support structure 2 between its first and second state. A seat member 3 is mounted to the first support structure 1 and is thereby displaceable in relation to the ground surface GS even when the second support structure 2 is in the second state with the friction creating members 4 pressing against the ground surface GS creating considerable friction against the ground surface GS. Considerable friction is to be understood as friction making the chair difficult to move along the ground surface GS, which could be compared with the moving of the chair using the wheels 6 adapted therefor, which is done much easier. According to one embodiment the considerable friction is created by the actuation device 5 pressing the friction creating members 4 against the floor with a force exceeding 100 Newton, according to another embodiment considerable friction is created by the actuation device 5 pressing the friction creating members 4 against the floor with a force exceeding 200 Newton and according to a third embodiment considerable friction is created by the actuation device 5 pressing the friction creating members 4 against the floor with a force exceeding 400 Newton. The required force is for example dependant on the material of the ground surface GS and/or the material of the friction creating members 4.

In an x-ray room a chair according to any of the embodiments herein could be used in the following way: The patient is seated in the chair; the personnel operating the x-ray room positions the chair in a suitable position for the x-ray device; the chair is braked against the ground surface by the friction creating members being pressed against the ground surface; the patient is further positioned using the displacement device and rotation device of the chair; a first x-ray image is created; the patient is repositioned using the displacement device and rotation device of the chair; a second x-ray image is created, this process continues until a satisfactory amount of images is created; friction creating members are elevated and the chair is rolled away to prepare next patient for x-ray.

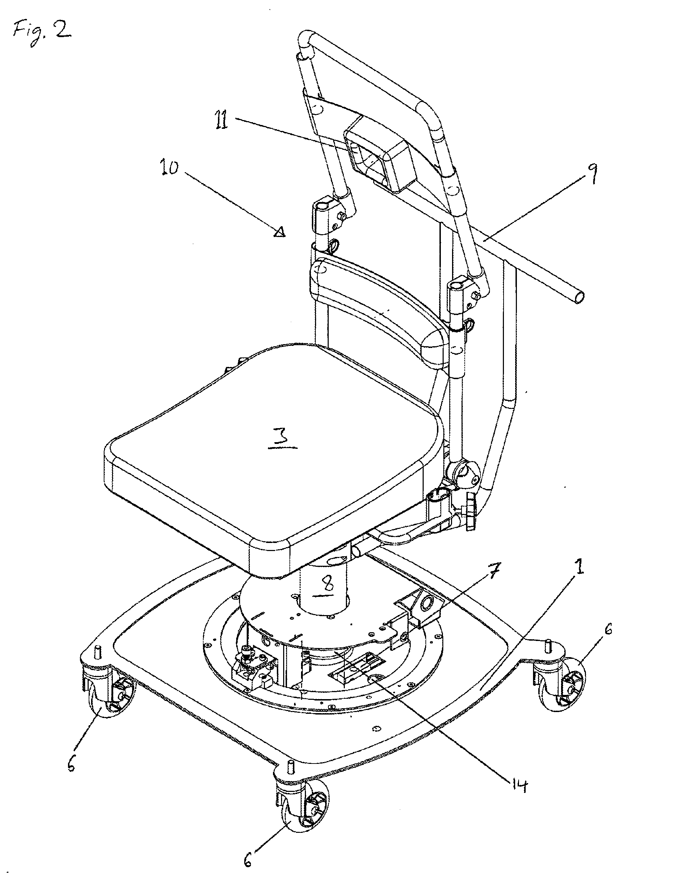

Fig. 2 shows a second embodiment of the chair in perspective view. The chair in fig. 2 comprises a first support structure 1 on which wheels 6 for making the chair movable in relation to the ground surface are mounted, the wheels 6 according to this embodiment being swiveling castors 6 which enables movement along all axes of the ground surface plane. A seat member 3 is mounted to a cylinder 8. The seat member 3 is by the connection with the cylinder 8 mounted to the first support structure 1. The mounting of the seat member 3 to the first support structure 1 could be a fixed fixation or a mounting enabling rotation and/or raising/lowering of the seat member 3. A displacement device 7 which connects the first support structure 1 to the second support structure (not visible) is visible through a hole in the first support structure 1. The displacement device 7 enables displacement of the first support structure 1 and thus the seat member 3 mounted thereon along two axes in relation to the second support structure. The displacement device 7 is in turn mounted to a bearing member 14 which enables rotation of the first support device 1 and thus the seat member 3 mounted thereon. The seat member 3 and the person seated thereon is thereby displaceable along two axis of the ground surface plane and rotatable preferably around an axis aligned with an axis of the spine of a person seated in the chair, which in x-ray use enable multiple images of the spine in different angles to be created. According to the embodiment of fig. 2, the seat member 3 further comprises a backrest 10 and a head rest 11. When using the chair when taking x-ray images it could be important to have the person seated in the chair in an exact position which could be obtained by leaning back against the back 10 and head rest 11. According to some embodiments (not shown) the head rest 11 further comprises a strap for further fixation of the head, which could be of particular importance when taking x-ray images of the upper back and neck. For x-ray use, the back 10 and headrest 11 needs to be of transradiant material such as carbon fiber or graphite for enabling the creation of x-ray images in a frontal-dorsal direction. The back rest could be tiltable or turnable to adapt the chair to the person sitting, to a person operating the chair, or to surrounding equipment. The functions adapted to customize the seat to a person or a use e.g. the rotation, the backrest and/or the raising and lowering of the seat could be mechanical, hydraulic, pneumatic or electric functions and could in some embodiments be adapted to be performed automatically.

The displacement device 7 is according to one embodiment equipped with brakes for braking or locking the displacement device 7 and thus the chair in specific positions, when the second support structure is in its second state. According to one embodiment the displacement device 7 and bearing member is locked per default and a releasing action is required by the person operating the chair in order to enable displacement and/or rotation of the chair. A control leaver 9 for controlling the displacement device 7 and bearing member 14 is provided, and according to the embodiment of fig. 2 attached to the cylinder 8. The control leaver 9 could comprise hand controls for the different brakes and also the control of the actuation device 5 actuating the friction creating members engaging the ground surface. When using the chair to create x-ray images of the person sitting in the chair the control leaver 9 needs to be removed, preferably be rotating the control leaver 9 around the cylinder 8 so that the leaver 9 is removed from the area of x-ray radiation.

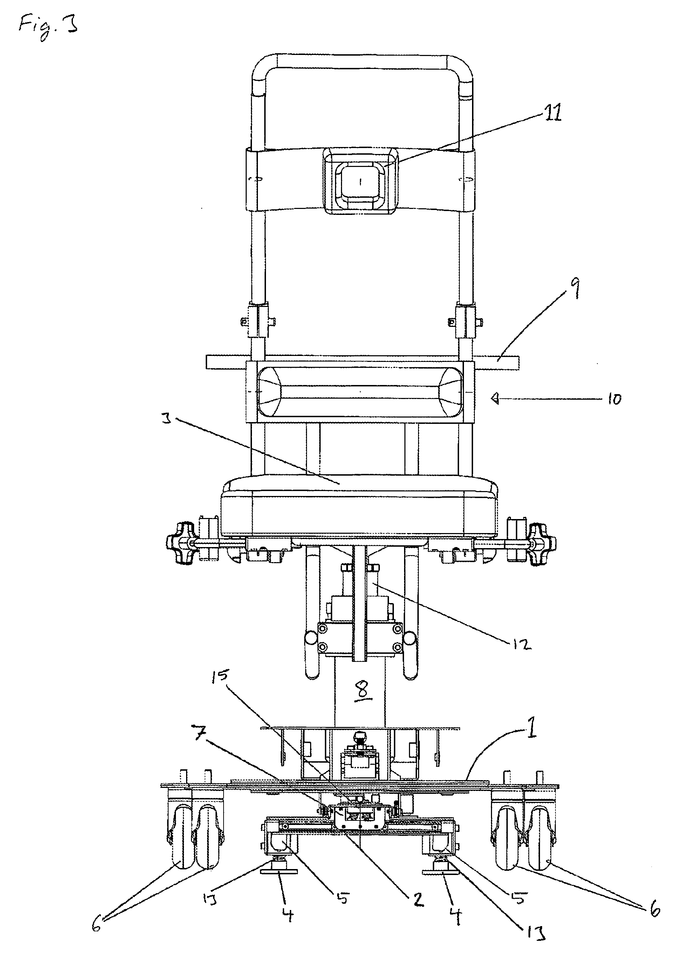

Fig. 3 shows the chair according to the embodiment of fig 2, in a frontal view. The first support structure 1 comprises the wheels 6, which are swiveling castors 6. The second support structure 2 is here visible comprising the actuators 5 which actuate the friction creating members 4 to engage the ground surface when the chair should be positioned. According to this embodiment the actuators 5 comprises springs 13 which are adapted to create a suitable pressure of the friction creating members 4 against the ground surface. According to this embodiment the actuators 5 have a mechanical construction which is manually controlled from a hand control mounted on the control leaver 9, the force from the hand control is then transferred through a wire to the actuators 5. However, according to other embodiments, the actuators 5 could be hydraulic, pneumatic or electric. The second support structure 2 is mounted to the displacement device 7 which enables displacement along two axes of the ground surface plane. According to this embodiment the displacement device 7 comprises a brake 15 which is braking the displacement device 7 per default, thus a releasing action is required to enable displacement of the seat member 3 in relation to the second support structure using the displacement device 7. The releasing action is according to this embodiment controlled from a manual hand control placed on the control leaver 9. However the brake 15 or the brakes for additional linear displacement or rotation could be assisted or replaced by a hydraulic, pneumatic or electric system. The seat member 3 with the back 10 and head rest 11 is also displayed in frontal view in fig. 3. The seat member 3 is supported by a actuation cylinder 12 which is mounted to the cylinder 8. The actuation cylinder 12 enables the raising and lowering of the chair to adapt the seat member 3 to different persons. According to some embodiments (not shown) the actuation cylinder 12 further enables a second point of rotation which for example could be used when the less accuracy is required.

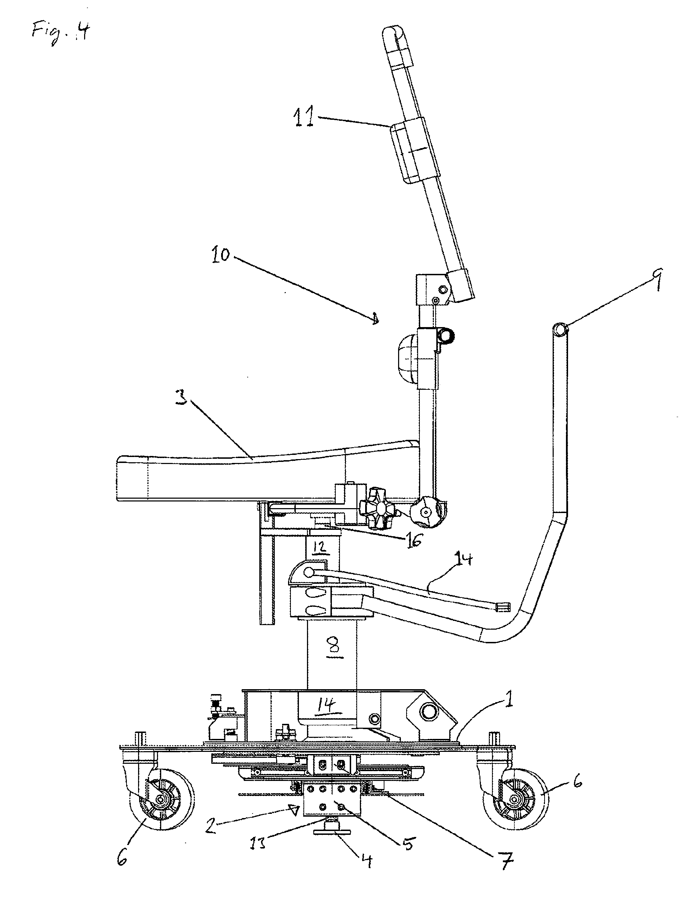

Fig. 4 shows the chair according to the embodiments of figs. 2 and 3 in a side view. The actuator 5 with the spring 13 actuating the friction creating member 4 is shown, being connected to the displacement device 7, which in turn is connected to the bearing member 14 enabling rotation of the first support structure 1 and thus the seat member 3 in relation of the second support structure 2. The view further shows the actuation cylinder 12 mounted on the cylinder 8. The actuation cylinder 12 comprises a piston 16 for raising and lowering the seat member 3. The actuation cylinder 12 is preferably a pneumatic or hydraulic cylinder 12 possible to operate from an operation leaver 14. The control leaver 9 comprising the hand controls for the actuators 5 and the brakes of the displacement 7 and rotation device is further shown. The control leaver 9 is possible to rotate around the cylinder 8 for moving the control leaver 9 when the chair is used in x-ray applications. The rotational function of the control leaver 9 comprises a lock function which needs to be manipulated before rotation of the leaver 9 is possible. This lock function enables a sturdy fixation of the leaver 9 when personnel operating the chair would like to use the leaver 9 to push or pull and thereby moving the entire chair.

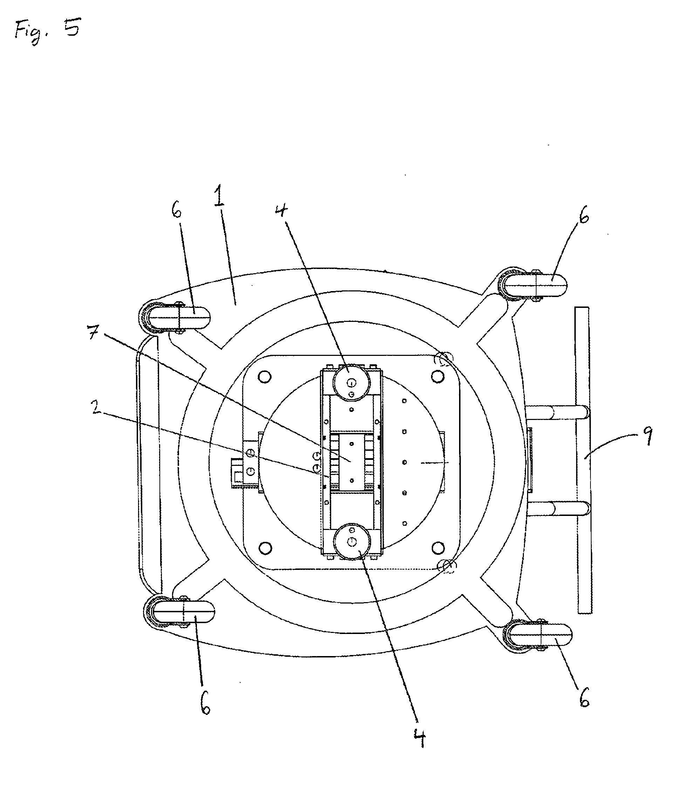

Fig. 5 shows the chair according to the embodiments of figs. 2, 3 and 4 from underneath clearly displaying the two friction creating members 4. According to the embodiments shown, the chair comprises two friction creating members 4, however it is equally conceivable that the chair comprises only one friction creating member or more than two friction creating members. The friction creating members 4 comprise a material adapted to create considerable friction against the ground surface thereby allowing the chair to be positioned without moving if no strong force is applied to the side of the chair. The material of the friction creating members is preferably some sort of rubber material; however this could be adapted to function with the particular ground surface. Fig. 5 further shows the friction creating members 4 being attached to the second support structure 2 which in turn is connected to the displacement device 7 displacing the second supporting device 2 in relation to the first supporting device 1. The fig. also shows the swiveling castors 6 attached to the first support structure 1 and the control lever 9 comprising the hand controls for the different mechanical functions.

The displacement device (pos. 7 in the different embodiments) could comprise an automatic return system comprising springs for enabling the displacement device to return to a reference position at the releasing of brakes and/or the manual releasing of a part of the chair. A similar functionality could also be built in to the rotation device, which enables the seat member to return to a rotational reference position at the releasing of the brakes and/or the manual releasing of a part of the chair.

To indicate the amount of displacement, the chair according to some embodiments comprises scales indicating the amount of linear displacement and/or the angle of rotation. This information could in x-ray applications be of interest to the person analyzing the x-ray images, it could also be of relevance if additional x-ray images is needed.

The x-ray application is herein described as an example of where the chair according to any of the embodiments herein could be used. However the inventive idea of the chair is advantageous in many different situations where a movable chair with an accurate displacement or positioning of the chair is needed, especially since chairs with swiveling castors have a tendency to be hard to position in a precise way. Examples of other possible areas of use could be for dental procedures, radiation treatments, magnetic radiology, computer tomography, and eye surgery.

## Claims

1. A chair for sitting, wherein said chair comprises:
• a first support structure (1) adapted to be movable in relation to the ground surface (GS), **characterized in that** said chair further comprises:
• a second support structure (2) adapted to have a first and second state, and
• a seat member (3) mounted to said first support structure (1), such that said seat member (3) is displaceable in relation to the ground surface (GS) when said second support structure (2) is in said second state, wherein:
o said second support structure (2), in said first state, is movable along with said first support structure (1),
o said second support structure (2), in said second state, is adapted to display considerable friction against the ground surface (GS), and wherein:
• said second support structure (2) is displacably mounted to said first support structure (1) by a displacement device (7) of said chair, wherein said first support structure (1) is connected to said displacement device (7) and said second support structure (2) is connected to said displacement device (7) and said displacement device (7) is adapted to enable linear movement of said first support structure (1) in relation to said second support structure (2) along a first axis substantially parallel to the ground surface.

2. The chair according to claim 1, wherein said first support structure (1) comprises wheels (6), and wherein said first support structure (1) is movable in relation to the ground surface (GS) using said wheels (6).

3. The chair according to any one of claims 1 and 2, wherein said second support structure (2) is adapted to display considerable friction against the ground surface (GS) by friction creating members (4) engaging the ground surface (GS) and thereby substantially fixating the second support structure (2) to a position on the ground surface (GS).

4. The chair according to any one of claims 1- 3, wherein the displacement device (7) is further adapted to enable linear movement of said first support structure (1) in relation to said second support structure (2) along a second axis.

5. The chair according to any one of the preceding claims, wherein the chair further comprises a rotation device adapted to enable rotation of the first support structure (1) in relation to the second support structure (2).

6. The chair according to claim 1 - 5, wherein said chair further comprises a brake adapted to brake any of: said linear movement of said displacement device along said first axis and/or said linear movement of said displacement device along said second axis and/or said rotational movement of said rotation device.

7. The chair according to any of the preceding claims, wherein said seat member (3) comprises a transradiant back rest (10).

8. The chair according to any one of the preceding claims, wherein said chair is adapted for x-ray imaging of a person seated in said chair.

9. The chair according to any one of claims 4 - 8, wherein said displacement device (7) comprises an automatic return system comprising springs for enabling the displacement device (7) to return to a reference position at the releasing of brakes and/or the manual releasing of a part of the chair.

10. The chair according to any one of claims 6 - 9, wherein said rotation device comprises an automatic return system which enables the seat member (3) to return to a rotational reference position at the releasing of the brakes and/or the manual releasing of a part of the chair.

11. The chair according to any one of the preceding claims, wherein the chair further comprises scales indicating the amount of linear displacement and/or the angle of rotation.

## Patentansprüche

1. Stuhl zum Sitzen, wobei der Stuhl Folgendes umfasst:
- eine erste Trägerstruktur (1), die daran angepasst ist, bezogen auf die Bodenfläche (GS) bewegbar zu sein, **dadurch gekennzeichnet, dass** der Stuhl ferner Folgendes umfasst:
- eine zweite Trägerstruktur (2), die daran angepasst ist, einen ersten und einen zweiten Zustand aufzuweisen, und
- ein Sitzelement (3), das an der ersten Trägerstruktur (1) derart befestigt ist, dass das Sitzelement (3) bezogen auf die Bodenfläche (GS) verschiebbar ist, wenn sich die zweite Trägerstruktur (2) in dem zweiten Zustand befindet, wobei:
- die zweite Trägerstruktur (2) in dem ersten Zustand gemeinsam mit der ersten Trägerstruktur (1) bewegbar ist,
- die zweite Trägerstruktur (2) in dem zweiten Zustand daran angepasst ist, beachtliche Reibung an der Bodenfläche (GS) zu zeigen, und wobei:
- die zweite Trägerstruktur (2) an der ersten Trägerstruktur (1) mit einer Verschiebevorrichtung (7) des Stuhls verschiebbar befestigt ist, wobei die erste Trägerstruktur (1) mit der Verschiebevorrichtung (7) verbunden ist und die zweite Trägerstruktur (2) mit der Verschiebevorrichtung (7) verbunden ist und die Verschiebevorrichtung (7) daran angepasst ist, eine lineare Bewegung der ersten Trägerstruktur (1) bezogen auf die zweite Trägerstruktur (2) entlang einer ersten Achse im Wesentlichen parallel zur Bodenfläche zu ermöglichen.

2. Stuhl nach Anspruch 1, wobei die erste Trägerstruktur (1) Räder (6) umfasst und wobei die erste Trägerstruktur (1) bezogen auf die Bodenfläche (GS) unter Verwendung der Räder (6) bewegbar ist.

3. Stuhl nach einem der Ansprüche 1 und 2, wobei die zweite Trägerstruktur (2) daran angepasst ist, beachtliche Reibung an der Bodenfläche (GS) zu zeigen, über Reibung erzeugende Elemente (4), die die Bodenfläche (GS) berühren und **dadurch** die zweite Trägerstruktur (2) an einer Stelle auf der Bodenfläche (GS) im Wesentlichen fixieren.

4. Stuhl nach einem der Ansprüche 1 bis 3, wobei die Verschiebevorrichtung (7) ferner daran angepasst ist, eine lineare Bewegung der ersten Trägerstruktur (1) bezogen auf die zweite Trägerstruktur (2) entlang einer zweiten Achse zu ermöglichen.

5. Stuhl nach einem der vorhergehenden Ansprüche, wobei der Stuhl ferner eine Drehvorrichtung umfasst, die daran angepasst ist, die Drehung der ersten Trägerstruktur (1) bezogen auf die zweite Trägerstruktur (2) zu ermöglichen.

6. Stuhl nach Anspruch 1 bis 5, wobei der Stuhl ferner eine Bremse umfasst, die daran angepasst ist, eins aus Folgendem zu bremsen: die lineare Bewegung der Verschiebevorrichtung entlang der ersten Achse und/oder die lineare Bewegung der Verschiebevorrichtung entlang der zweiten Achse und/oder die Drehbewegung der Drehvorrichtung.

7. Stuhl nach einem der vorhergehenden Ansprüche, wobei das Sitzelement (3) eine röntgenstrahlenundurchlässige Rückenlehne (10) umfasst.

8. Stuhl nach einem der vorhergehenden Ansprüche, wobei der Stuhl zum Röntgen einer Person, die in dem Stuhl sitzt, geeignet ist.

9. Stuhl nach einem der Ansprüche 4 bis 8, wobei die Verschiebevorrichtung (7) ein automatisches Rückstellsystem umfasst, das Federn umfasst, damit die Verschiebevorrichtung (7) beim Lösen der Bremsen und/oder dem manuellen Lösen eines Teils des Stuhls in eine Bezugsposition zurückkehren kann.

10. Stuhl nach einem der Ansprüche 6 bis 9, wobei die Drehvorrichtung ein automatisches Rückstellsystem umfasst, das es ermöglicht, dass das Sitzelement (3) beim Lösen der Bremsen und/oder dem manuellen Lösen eines Teils des Stuhls in eine Drehbezugsposition zurückkehrt.

11. Stuhl nach einem der vorhergehenden Ansprüche, wobei der Stuhl ferner eine Maßeinteilung umfasst, die den Wert der linearen Verschiebung und/oder den Drehwinkel anzeigt.

## Revendications

1. Chaise pour s'asseoir, ladite chaise comprenant :
• une première structure de support (1) adaptée pour être mobile par rapport à la surface du sol (GS), **caractérisée en ce que** ladite chaise comprend en outre :
• une seconde structure de support (2) adaptée pour présenter un premier et un second état, et
• un élément d'assise (3) monté sur ladite première structure de support (1), de sorte que ledit élément d'assise (3) est déplaçable par rapport à la surface du sol (GS) quand ladite seconde structure de support (2) est dans ledit second état, dans laquelle :
o ladite seconde structure de support (2), dans ledit premier état, est mobile ensemble avec ladite première structure de support (1),
o ladite seconde structure de support (2), dans ledit second état, est adaptée pour présenter un frottement considérable contre la surface du sol (GS), et dans laquelle :
• ladite seconde structure de support (2) est montée de manière déplaçable sur ladite première structure de support (1) par un dispositif de déplacement (7) de ladite chaise, dans laquelle ladite première structure de support (1) est connectée audit dispositif de déplacement (7) et ladite seconde structure de support (2) est connectée audit dispositif de déplacement (7) et ledit dispositif de déplacement (7) est adapté pour permettre un mouvement linéaire de ladite première structure de support (1) par rapport à ladite seconde structure de support (2) le long d'un premier axe essentiellement parallèle à la surface du sol.

2. Chaise selon la revendication 1, dans laquelle ladite première structure de support (1) comprend des roues (6), et dans laquelle ladite première structure de support (1) est mobile par rapport à la surface du sol (GS) en utilisant lesdites roues (6).

3. Chaise selon l'une quelconque des revendications 1 et 2, dans laquelle ladite seconde structure de support (2) est adaptée pour présenter un frottement considérable contre la surface du sol (GS) par des éléments (4) créant un frottement venant en contact avec la surface du sol (GS) et fixant ainsi essentiellement la seconde structure de support (2) dans une position sur la surface du sol (GS).

4. Chaise selon l'une quelconque des revendications 1 à 3, dans laquelle le dispositif de déplacement (7) est en outre adapté pour permettre un mouvement linéaire de ladite première structure de support (1) par rapport à ladite seconde structure de support (2) le long d'un second axe.

5. Chaise selon l'une quelconque des revendications précédentes, dans laquelle la chaise comprend en outre un dispositif de rotation adapté pour permettre une rotation de la première structure de support (1) par rapport à la seconde structure de support (2).

6. Chaise selon les revendications 1 à 5, dans laquelle ladite chaise comprend en outre un frein adapté pour freiner l'un quelconque parmi : ledit mouvement linéaire dudit dispositif de déplacement le long dudit premier axe et/ou ledit mouvement linéaire dudit dispositif de déplacement le long dudit second axe et/ou ledit mouvement de rotation dudit dispositif de rotation.

7. Chaise selon l'une quelconque des revendications précédentes, dans laquelle ledit élément d'assise (3) comprend un dossier radiotransparent (10).

8. Chaise selon l'une quelconque des revendications précédentes, dans laquelle ladite chaise est adaptée pour la radiographie d'une personne assise dans ladite chaise.

9. Chaise selon l'une quelconque des revendications 4 à 8, dans laquelle ledit dispositif de déplacement (7) comprend un système de retour automatique comprenant des ressorts pour permettre au dispositif de déplacement (7) de revenir à une position de référence lors du déblocage des freins et/ou du déblocage manuel d'une pièce de la chaise.

10. Chaise selon l'une quelconque des revendications 6 à 9, dans laquelle ledit dispositif de rotation comprend un système de retour automatique qui permet à l'élément d'assise (3) de revenir à une position de référence rotationnelle lors du déblocage des freins et/ou du déblocage manuel d'une pièce de la chaise.

11. Chaise selon l'une quelconque des revendications précédentes, dans laquelle la chaise comprend en outre des graduations indiquant la quantité de déplacement linéaire et/ou l'angle de rotation.
